# EUROPEAN PATENT APPLICATION

(11) **EP 1 454 641 A2**
(43) Date of publication of application: **08.09.2004**
(21) Application number: 04250706.1
(22) Date of filing: 10.02.2004
(51) Int. Cl.: A61L 27/38, A61L 27/56

(54) **Porous implantable medical device seeded with mammalian cells**

(30) Priority: 11.02.2003 US 364030
(71) Applicant: ETHICON, INC., Somerville, New Jersey 08876 (US)
(72) Inventor: Rezania, Alireza, Hillsborough NJ 08844 (US); Zimmerman, Mark C., East Brunswick NJ 08816 (US); Ten-Huisen, Kevor S., Clinton NJ 08809 (US)
(74) Representative: Mercer, Christopher Paul

(57) **Abstract**

The present invention relates to an implantable medical device containing a porous scaffold which is seeded with at least one mammalian cell other than immune cells and which is disposed within the device, which device is suitable for the treatment of a mammalian disease, more specifically, diabetes mellitus.

## Description

### FIELD OF THE INVENTION

The present invention relates to an implantable medical device containing a porous scaffold which is seeded with at least one mammalian cell other than immune cells and which is disposed within the device, which device is suitable for the treatment of a mammalian disease, more specifically, diabetes mellitus.

### BACKGROUND OF THE INVENTION

There is a clinical need for biocompatible and biodegradable structural matrices that facilitate tissue infiltration to repair/regenerate diseased tissue or to facilitate delivery of cells to a defect site or damaged organ. Thus far, previous attempts at using biodegradable scaffolds to repair tissue or restore organ function have proven insufficient to substantially alter or accelerate the repair process. There are many diseases, such as diabetes mellitus (DM), which result from an absence of a bioactive factor secreted by cells resident in the tissue. DM results from destruction of beta cells (Type I) in the pancreas or from insensitivity of muscle or adipose tissues to the hormone insulin (Type II). Current methods of treatment include diet and exercise, oral hypoglycemic agents, insulin injections, insulin pump therapy and whole pancreas or islet transplantation. The most common treatment involves daily injections of endogenous source such as porcine, bovine or human insulin. Another treatment approach has been transplantation of whole pancreas organ. Transplanting a whole adult pancreas is a major, technically complex operation that also requires aggressive immunosuppressive drugs. Furthermore, the limited availability of cadaver pancreas restricts the widespread use of this approach. There are many advantages of cellular over whole pancreas transplantation, including lower tissue mass, less invasive therapy, access to immunomanipulation and engineering of graft composition. However, until recently, islet grafting has been generally unsuccessful due to aggressive immune rejection of islets. Recent reports indicate that a glucocorticoid-free immunosuppressive regimen can significantly benefit the patients with brittle type I diabetes. However, the patients using this treatment are prone to renal complications and mouth ulcers and require large number of islets (~9000 islet equivalents/kg) to induce normoglycemia. Thus, there has been an intense effort to devise islet cell transplantation strategies that avoid the large doses of immunesuppressive drugs and that use a commercially viable islet cell source. This has led to the concept of immuno-isolation via microencapsulation, which involves shielding of the islets with a selectively permeable membrane. The membrane allows passage of small molecules, such as nutrients, oxygen, glucose and insulin, while restricting the passage of larger humoral immune molecules and immune cells. In theory, one could use an immune-isolation device with an abundant animal islet cell source, such as porcine, to treat DM. However, in practice this approach has had little success in large animal models or in the clinic due to fibrosis of the device, limited oxygen supply within the device, and passage of small humoral immune molecules that lead to islet loss.

An alternative approach to immune-isolation is the creation of an immunologically privileged site by transplanting Sertoli cells into a nontesticular site in a mammal. This site allows for subsequent transplantation of islets that produce insulin. The immune privileged site would allow transplantation of either human or animal derived islets. One of the drawbacks of this approach is that the transplanted Sertoli cells and islets are not physically restricted to the site of transplantation. This can lead to migration of these cells to unwanted tissue sites and ultimately to the loss of islets. Furthermore, the immune privileged environment created by Sertoli cells is most effective when the islets are in close vicinity of the Sertoli cells.

### SUMMARY OF THE INVENTION

The present invention is directed to an implantable medical device that is suitable for use in the treatment of disease or in the repair or regeneration of tissue of a mammal, comprising a shell having an outer surface and an interior lumen and a biocompatible, porous scaffold comprising a plurality of at least one mammalian cell type other than an immune cell seeded therein, and where the scaffold is disposed within the interior lumen. The invention also is directed to methods of treatment of disease or in the repair or regeneration of tissue in mammal, comprising implanting the device of the present invention in the mammal.

### BRIEF DESCRIPTION OF THE FIGURES

Figure 1 is a perspective drawing of one embodiment of the implantable device described herein.
Figure 2 is a scanning electron micrograph of one embodiment of a textured fiber scaffold suitable for use in the present invention made by the process described in Example 1.
Figure 3 is a scanning electron micrograph of one embodiment of a foam scaffold suitable for use in the present invention made by the process described in Example 4.
Figure 4 is a scanning electron micrograph of one embodiment of a nonwoven scaffold suitable for use in the present invention made by the process described in Example 5.
Figure 5 shows a H&E section (40X) of Sertoli cells seeded on the device described in Example 6.

### DETAILED DESCRIPTION OF THE INVENTION

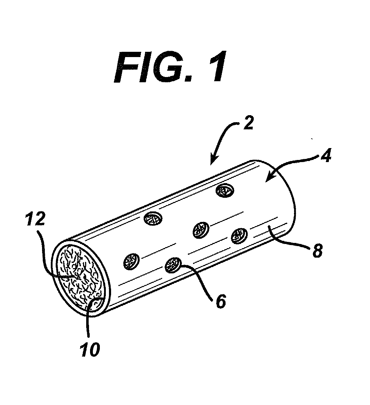

An implantable device comprising a tissue scaffold is disclosed herein which is used in the treatment of a disease or in the repair and regeneration of mammalian tissue. A perspective view of the implantable device is provided in Figure 1. The implantable device 2 is comprised of a shell 4 surrounding an interior lumen 10. The shell 4 preferably has pores 6 that extend from the outer surface 8 to the interior lumen 10. The interior lumen will have a volume of at least 1 x 10⁻⁸ cm³, preferably at least 3 x 10⁻⁸ cm³. The shell 4 may have a variety of three-dimensional shapes (e.g. cylindrical, spherical, rectangular, rhomboidal, star-shaped, etc.). For example, the shell 4 will generally have a longitudinal axis and a cross-section that may be circular, oval or polygonal. Preferred for ease of manufacture is a cylindrical shape. A cylindrically-shaped implantable device 2 is illustrated in Figure 1. The ends of the cylindrically-shaped implantable device may be capped or left open as illustrated in Figure 1. The outer surface 8 of the implantable device 2 has numerous pores 6 that allow for the ingress of nutrients and egress of cellular wastes. The surface area of the pores 6 will generally comprise up to about 95 percent of the surface area of the outer surface. The pores 6 size may range from about 0.1 to about 500 microns. The interior 10 of the implantable device 2 comprises scaffold 12. Scaffold 12 comprises a plurality of biocompatible fibers (e.g. yarn or tow), a porous biocampatable foam, or a combination of the two.

The fibrous scaffold is made from biocompatible fibers, preferably textured fibers that provide a much lower bulk density filling than non-texturized fiber. The low bulk density of textured fibers enables implantation of a significant numbers of cells and retention of the fibrous scaffold 12 within the shell 4. The porous foam matrix is preferably elastomeric, with pore size in the range of 5-200 microns. The scaffold 12 is loaded with cells or cellular matter and, optionally, other biologically active or pharmaceutically active compounds, attachment factors, genes, peptides, proteins, nucleotides, carbohydrates or synthetic molecules, depending on the application.

The shell 4 and the scaffold 12 of the implantable device 2 will comprise a biocompatible material that may be absorbable or non-absorbable. The device preferably comprises biocompatible materials that are flexible, thereby minimizing irritation to the patient. Preferably, the shell will comprise polymers or polymer blends having glass transition temperature below physiologic temperature. Alternatively, the device may comprise a polymer blended with a plasticizer that makes it flexible.

Numerous biocompatible absorbable and nonabsorbable materials can be used to make the shell or scaffold. Suitable nonabsorbable materials for use in the manufacture of the shell or scaffold include, but are not limited to, polyamides, polyesters (e.g. polyethylene terephthalate, polybutyl terphthalate, copolymers and blends thereof), fluoropolymers (e.g. polytetrafluoroethylene and polyvinylidene fluoride, copolymers and blends thereof), polyolefins, organosiloxanes (e.g. polydimethylsiloxane rubber such as SILASTIC® silicone tubing from Dow Coming), polyvinyl resins (e.g. polystyrene, polyvinylpyrrolidone, etc.), and blends thereof.

Additionaly, scaffold 12 may comprise naturally-derived biopolymers. As used herein, the term "biopolymer" is understood to include, but is not intended to be limited to, hyaluronic acid (HA), animal-derived collagen, recombinant collagen, elastin, alginates, chondroitin sulfate, chitosan, small intestine submucosa (SIS) and blends thereof. These biopolymer scaffolds can be further modified to enhance their mechanical or degradation properties by introducing cross-linking agents or changing the hydrophobicity of the side residues. The biopolymer may be lyophilized inside the lumen of the device or injected directly into the lumen of the device.

Polyesters are also well known commercially available synthetic polymers that may be used to make the shell or scaffold. The most preferred polyester for making this device is polyethylene terephthalate. Generally, polyethylene terephthalate polymers used to make fibers will have a weight average molecular weight (M_{w}) of greater than 30,000(M_{w}), preferably greater than 40,000(M_{w}), most preferably in the range of from about 42,000(M_{w}) to about 45,000(M_{w}). The filaments formed from these polymers should have a tenacity of greater than 5 grams/denier, preferably greater than 7 grams/denier. Polyethylene terephthalate yams are commonly available from a variety of commercial fiber suppliers, such as E.I. DuPont and Hoechst Celanese. Preferred are commercially available fibers that may be purchased from Hoechst Celanese under the trademark TREVIRA® High Tenacity type 712 and 787 polyester yarns.

A variety of fluoropolymers may also be used to make the shell and the scaffolds, such as polytetrafluoroethylene and polyvinylidene fluoride, copolymers and blends thereof. Currently, preferred are blends of polyvinylidene fluoride homopolymer, and copolymers of polyvinylidene fluoride and hexafluoropropylene.

As previously stated, the term polypropylene for the purposes of this application include atactic, but will be preferably isotactic and syndiotactic polypropylene, and blends thereof, as well as, blends composed predominantly of isotactic or syndiotactic polypropylene blended with heterotactic polypropylene and polyethylene and copolymers composed predominantly of propylene and other alpha-olefins, such as ethylene. The preferred polypropylene material for making fibers is isotactic polypropylene without any other polymers blended or monomers copolymerized therein. The preferred method for preparing the flexible polypropylene fibers of the present invention utilizes as the raw material pellets of isotactic polypropylene homopolymer having a (M_{w}) of from about 260,000(M_{w}) to about 420,000(M_{w}). Polypropylene of the desired grade is commercially available in both powder and pellet form.

A variety of bioabsorbable polymers can be used to make the shell or scaffold of the present invention. Examples of suitable biocompatible, bioabsorbable polymers include, but are not limited to, polymers selected from the group consisting of aliphatic polyesters, poly(amino acids), copoly(ether-esters), polyalkylenes oxalates, polyamides, tyrosine derived polycarbonates, poly(iminocarbonates), polyorthoesters, polyoxaesters, polyamidoesters, polyoxaesters containing amine groups, poly(anhydrides), polyphosphazenes, biopolymers such as collagen, elastin, bioabsorbable starches, and blends thereof.

Particularly well suited for use in the present invention are biocompatible absorbable polymers selected from the group consisting of aliphatic polyesters, copolymers and blends which include, but are not limited to, homopolymers and copolymers of lactide, including D-, L-, lactic acid and D-, L- and meso lactide), glycolide, including glycolic acid, epsilon-caprolactone, para-dioxanone, alkyl-substituted derivatives of para-dioxanone (e.g. 6,6-dimethyl-1,4-dioxan-2-one, trimethylene carbonate, alkyl-substituted derivatives of 1,3-dioxanone, delta-valerolactone, beta-butyrolactone, gamma-butyrolactone, epsilon-decalactone, hydroxybutyrate, hydroxyvalerate, 1,4-dioxepan-2-one and its dimer 1,5,8,12-tetraoxacyclotetradecane-7,14-dione, 1,5-dioxepan-2-one, and polymer blends thereof.

Preferred fibers are made from lactide and glycolide, sometimes referred to herein as simply homopolymers and copolymers of lactide and glycolide, and copolymers of glycolide and epsilon-caprolactone. More preferred for use as a fiber is a copolymer that is made with from about 80 weight percent to about 100 weight percent glycolide, with the remainder being lactide. Most preferred are copolymers of from about 85 to about 95 weight percent glycolide, with the remainder being lactide.

Preferred foam scaffolds may be composed of homopolymers, as well as copolymers or blends of glycolide, lactide, polydioxanone and epsilon-caproloactone. More preferred are copolymers of glycolide and epsilon-caprolactone. Most preferred is a 65/35 glycolide/epsilon-caprolactone copolymer.

As used herein, the term "glycolide" is understood to include polyglycolic acid. Further, the term "lactide" is understood to include L-lactide, D-lactide, blends thereof, and lactic acid polymers and copolymers.

The molecular weight (M_{w}) of the polymers used in the present invention can be varied, as is well know in the art, to provide the desired performance characteristics. However, it is preferred to have aliphatic polyesters having a molecular weight (M_{w}) that provides an inherent viscosity between about 0.5 to about 5.0 deciliters per gram (dl/g) as measured in a 0.1 g/dl solution of hexafluoroisopropanol at 25 °C, and preferably between about 0.7 and 3.5 deciliters per gram (dl/g).

As mentioned above, the outer surface 8 of shell 4 preferably will be perforated with pores 6, which provide a passageway for the ingress of nutrients and egress of cellular wastes to the interior lumen 10 of the implantable device 2. The shell 4 is preferably made from one or more absorbable polymers that may become more permeable to aqueous media as they degrade. Absorbable polymers can either be of natural or synthetic origin. The absorbable polymers for the shell most preferably have a glass transition temperature below physiologic temperature and would therefore be less irritating when implanted in soft tissues. Preferred polymers for the shell would include copolymers with a significant content (at least 30 weight percent) of epsilon-caprolactone or para-dioxanone. A particularly desirable composition includes an elastomeric copolymer of from about 35 to about 45 weight percent epsilon-caprolactone and from about 55 to about 65 weight percent glycolide, lactide (or lactic acid), and mixtures thereof. Another particularly desirable composition includes para-dioxanone homopolymer or copolymers containing from about 0 to about 80 weight percent para-dioxanone and from about 0 to about 20 weight percent of either lactide or glycolide, and combinations thereof.

The shell 4 can be of any shape into which the scaffold can be placed. The shell can initially have openings that may be sealed following placement of the scaffold 12. The shell 4 can be made by conventional polymer processing techniques, including molding, welding, casting, extrusion, injection molding, machining process, or combinations thereof. These conventional procedures are well known in the art and described in the Encyclopedia of Polymer Science and Engineering, incorporated herein by reference. Melt extrusion is the preferred method of processing, as it is rapid, inexpensive, scalable and can be performed solvent-free for many polymers of interest. Processing aides and plasticizers can be added to the polymer to decrease the processing temperature and/or modify the physical properties of the construct. Processing aides, such as solvents, can be added to decrease the processing temperature by decreasing the glass transition temperature of the polymer. Subsequently, the aide can be removed by either heat and/or vacuum or by passing the extruded construct through a secondary solvent in which the polymer has minimal solubility but is miscible with the processing aide. For example, halogenated solvents such as methylene chloride or chloroform can be added to homo- and copolymers of lactide and epsilon-caprolactone. After extrusion, the solvent can be removed through evaporation, vacuum, and/or heat. These solvents could also be extracted by passing the extrudate through a secondary solvent such as alcohol, which has miscibility with the halogenated solvent. Plasticizers can also be incorporated into a polymer to increase its workability, flexibility or distensibility. Typically these materials work by increasing the free volume of the polymer. For example, many citrates, malates and caprilates will work to plasticize many aliphatic polyesters. Oligomers of a given polymer or copolymer can also be used to plasticize a system.

The pores 6 in the shell 4 generally are large enough to provide for the ingress of nutrients and egress of cellular wastes. The pores are preferably larger than about 0.1 microns but smaller than about 500 microns in cross-sectional diameter. The cross-sectional area of pores 6 comprise up to about 95 percent of the outer surface of device 2. The pores can be formed using any appropriate drilling technique (e.g. using a hypodermic needle, mechanical or laser) or alternatively by including a solvent or water-soluble solid in the wall polymer which later can be leached out by immersing the tube in the solvent to generate the hole. Alternatively, if biocompatible water-soluble particles such as sugars, amino acids, polymers such as PVP, proteins such as gelatin, carbohydrates such as hyalyronic acid and certain carboxy methylcelluloses are used, the device can be implanted with the particles present. Upon exposure to body fluids the pore-forming particles can leach out or degrade, thus forming pores. Most of the pore must extend completely through the wall of the device and provide a pathway for nutrient ingress into and cellular waste egress from the interior lumen 10 of device 2. If the implantable device 2 has one or more open ends, the open ends of the implantable device can either be sealed or left open, but are preferably left open.

In another embodiment of the present invention, at least one end of the implantable device is partially or totally filled with a biocompatible absorbable or non-resorbable plug. At a later time point, such as after implantation of device 2, the plug can be removed, and a scaffold, with or without cells, can be inserted into the open end of device 2. Optionally, the removed plug, or a new plug, can be inserted into the open end of device 2.

Fibers suitable for use in the present device can be made using conventional spinning processes, such as melt spinning processes or solution spinning. After spinning, the yams may be quenched, treated with a spin finish, drawn and annealed as is known in the art. The fibrous scaffold made from these fibers should have a porosity of greater than 20%, more preferably from about 25% to about 95%, and most preferably from about 30% to about 90%.

The fibrous scaffold should be made up of filaments having a denier in the range of from about 0.2 to about 10, preferably from about 0.8 to about 6, more preferably from about 1 to about 3. The filaments are commonly extruded in bundles (yams) having a denier in the range of from about 20 to about 400 denier, preferably from about 50 to about 100 denier. The fibers need to be treated to develop the bulk density or porosity needed for a fibrous scaffold. The preferred yams for this application are textured yams. There are many forms of textured yams that may be used to form a fibrous scaffold, such as bulked yams, coil yams, core bulked yams, crinkle yams, entangled yams, modified stretch yams, nontorqued yams, set yams, stretch yams and torqued yams, and combinations thereof. Methods for making these yams are well known and include the false-twisted method, entanglement (e.g. rotoset or air jet entangled), crimping (e.g. gear crimped, edge crimped or stuffer box crimped), and knit-de-knit. Preferably the fibers will be textured by a false-twisting method, the stuffer box method or knit-de-knit method of textile texturing. The filaments are texturized to provide a high degree of permanent crimping or random looping or coiling. Crimped fibers are currently preferred. Crimping causes the orientation of the filament to change angle at the crimping points. The angle change is preferably greater than 10 degrees at each crimp point. The crimping can be accomplished through a variety of processes, but is most easily generated by feeding the extruded filaments through a stuffer box.

The fibrous scaffold is preferably a texturized fiber made from an absorbable polymer that can either be of natural or synthetic origin. Each fiber filament preferably has a diameter of less than 20 microns, most preferably less than 15 microns. This imparts to the filaments sufficient flexibility to completely fill the lumen of the tube and provide a suitable surface for cells to colonize in the lumen of the shell. The fibers preferably will take longer than 1 month to biodegrade (via hydrolysis and/or enzymatic activity) in a normal subcutaneous implantation, but will be completely biodegraded within 6 months, more preferably between 1 and 4 months. An example of a good polymer for making a fibrous scaffold is a copolymer of 90% glycolide (or glycolic acid) and 10% lactide (or lactic acid) having an inherent viscosity between about 0.7 to about 1.5 deciliters per gram (dl/g), as measured in a 0.1 g/dl solution of hexafluoroisopropanol at 25°C.

The most significant advantage with the use of fibrous scaffold is that the fibers can be easily placed within the shell. For example, a textured fiber can be stretched and then the shell extruded, molded or otherwise coated or shaped around them. Following placement of the shell around the stretched fibers, the tension can be relaxed, which allows the fibers to assume their crimped shapes and fill the space inside the shell. The textured fibers can be wound onto spools in very long lengths, which can be continuously fed as a core in a core-sheath or wire coating extrusion process. The sheath can be a molten polymer that is co-extruded and drawn with the stretched fibers. Individual units could be created by cutting the core sheath constructs to a desired length. Perforations can be created by piercing the tubing wall to form small holes.

Small bunches of fibers can be stretched, compressed or otherwise exposed to robust mechanical processing. This is an important consideration in miniaturization of the device. Formation of sub-millimeter devices necessarily subjects the filling to significant stresses in order to fit within the small dimensions of the shell. Miniaturization is very important in minimizing patient pain and discomfort following implantation of the device. Hence the use of fibers, which can be compressed, enables a smaller device which is preferable from the patient's standpoint.

At first glance it may appear desirable to fill the shell with simple straight fibers. However, straight fibers would settle and bunch in the shell over time and would not provide a hospitable environment for ingress of large numbers of cells. Additionally, straight fiber would require that the device be modified to prevent the fibers from falling out of the device during handling. If the fibers were densely packed or braided, so as to provide an interference fit in the shell, there would not be sufficient porosity for cell colonization. Texturizing the fibers allows them to effectively fill space while maintaining porosities needed for colonization with high cell number densities. This low bulk density property of the texturized fibers enables an interference fit with the walls of the shell without having to worry about compaction of the filling during storage and handling.

The textured fibers can either be filled into a preformed tube or the tube can be extruded around the filaments. During the filling process it may be desirable to stretch the filaments to a straight orientation. This radially compresses the fibers to a much smaller diameter than they occupy when in a relaxed state. The void volume in the lumen of the tube is preferably greater than 30%, more preferably greater than 50%. Once relaxed the textured filaments should completely fill the lumen of the device and should stay in place in the lumen due to the compressive force exerted by the tubing walls on the filling.

A preferred process for generating the textured fiber filled tubes consists of extruding the tubing around the stretched filaments in a continuous manner. This can be accomplished by having the textured fiber wound on a spool and fed under tension through the lumen of an extruder die as a core around which a sheath of wall polymer is continuously extruded. Perforations can later be drilled through the wall of the polymer either mechanically or using electromagnetic radiation (e.g. laser ablation). It is especially desirable to adjust the depth of drilling so that the wall is completely punctured, although the filling is not damaged. With electromagnetic radiation this can be accomplished by providing just enough focused energy to ablate through the wall of the tube. Alternatively, it is possible to fill a preformed tube by tying the textured fiber to a thin wire or needle and then dragging the textured filaments under tension through the tubing. Additionally, it is possible to fill a preformed tube by using a pressure differential (e.g. vacuum or blown air) to pull the textured filament through the tubing. In this configuration the perforations in the tube can be created either pre or post filling of the lumen. The length of the textured fiber filled tube is cut to be greater than a few millimeters and more preferably greater than 5 millimeters.

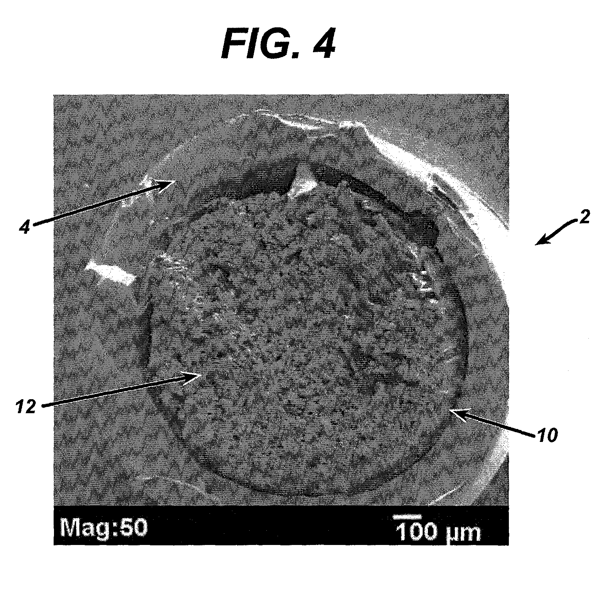

Alternatively, the scaffold 12 of the present invention can comprise a nonwoven fibrous scaffold. Figure 4 shows implantable device 2, comprised of shell 4 surrounding interior lumen 10. Lumen 10 of implantable 2 is filled with scaffold 12 in the form of nonwoven fibers.

The nonwoven scaffold can be fabricated using wet-lay or dry-lay fabrication techniques. Fusing the fibers of the nonwoven scaffold of the implantable device with another biodegradable polymer, using a thermal process, can further enhance the structural integrity of the fibrous nonwoven scaffold of the implantable device. For example, bioabsorbable thermoplastic polymer or copolymer, such as epsilon-polycaprolactone (PCL) in powder form, may be added to the nonwoven scaffold, followed by a mild heat treatment that melts the PCL particles, while not affecting the structure of the fibers. This powder possesses a low melting temperature and acts as a binding agent later in the process to increase the tensile strength and shear strength of the nonwoven scaffold. The preferred particulate powder size of PCL is in the range of 10-500 □m in diameter, more preferably 10-150 □m in diameter. Additional binding agents include a biodegradable polymeric binders selected from the group consisting of polylactic acid, polydioxanone, polyglycolic acid, or combinations thereof.

Alternatively, the fibers in the nonwoven scaffold may be fused together by spraying or dip coating the nonwoven scaffold in a solution of another biodegradable polymer.

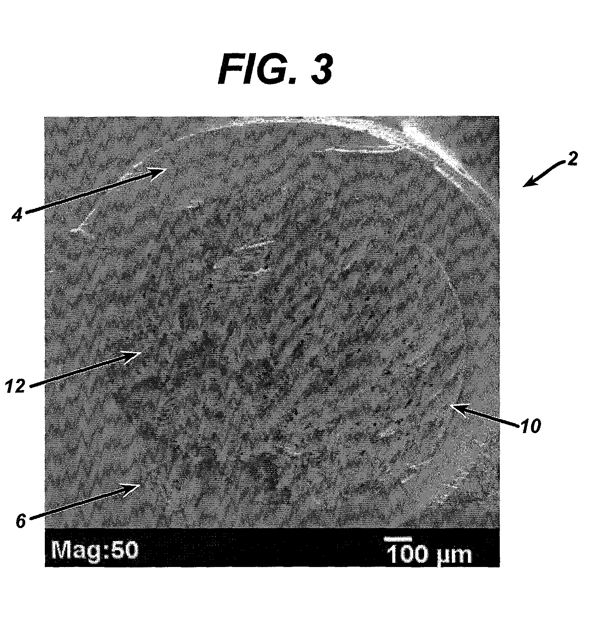

Scaffold 12 located in the lumen of implantable device 2 of the present invention may alternatively comprise a biocompatible foam. Figure 3 shows implantable device 2, comprised of shell 4 surrounding interior lumen 10. Lumen 10 of implantable device 2 is filled with scaffold 12 in the form of a porous foam.

The foam scaffold may be formed by a variety of techniques well known to those having ordinary skill in the art. For example, the polymeric starting materials may be foamed by lyophilization, supercritical solvent foaming, gas injection extrusion, gas injection molding or casting with an extractable material (e.g., salts, sugar or similar suitable materials).

In one embodiment, the foam scaffold of the implantable device may be made by a polymer-solvent phase separation technique, such as lyophilization. Generally, however, a polymer solution can be separated into two phases by any one of the four techniques: (a) thermally induced gelation/crystallization; (b) non-solvent induced separation of solvent and polymer phases; (c) chemically induced phase separation, and (d) thermally induced spinodal decomposition. The polymer solution is separated in a controlled manner into either two distinct phases or two bicontinuous phases. Subsequent removal of the solvent phase usually leaves a porous structure of density less than the bulk polymer and pores in the micrometer ranges.

The steps involved in the preparation of the foam scaffold include choosing the appropriate solvents for the polymers to be lyophilized and preparing a homogeneous solution of the polymer in the solution. The polymer solution then is subjected to a freezing and a vacuum drying cycle. The freezing step phase-separates the polymer solution and the vacuum drying step removes the solvent by sublimation and/or drying, thus leaving a porous polymer structure, or an interconnected open-cell porous foam.

Suitable solvents that may be used in the preparation of the foam scaffold component include, but are not limited to, tetrahydrofuran (THF), dimethylene fluoride (DMF), and polydioxanone (PDO), acetone, acetates of C2 to C5 alcohols (e.g., ethyl acetate and t-butylacetate), 1,4-dioxane, 1,3-dioxolane, 1,3-dioxolane-2-one (ethylene carbonate), dimethlycarbonate, benzene, toluene, benzyl alcohol, p-xylene, N-methyl pyrrolidone, dimethylformamide, chloroform, 1,2-dichloromethane, dimethylsulfoxide and mixtures thereof. Among these solvents, a preferred solvent is 1,4-dioxane. A homogeneous solution of the polymer in the solvent is prepared using standard techniques.

The applicable polymer concentration or amount of solvent that may be utilized will vary with each system. Generally, the amount of polymer in the solution can vary from about 0.5% to about 90% by weight and, preferably, will vary from about 0.5% to about 30% by weight, depending on factors such as the solubility of the polymer in a given solvent and the final properties desired in the foam scaffold.

The cellular matter loaded into the tissue scaffold device may be mammalian cells isolated from vascular or avascular tissues, depending on the application or the disease. Furthermore, the cells may be cultured under standard culture conditions to expand the number of cells followed by removal of the cells from culture plates and administering into the scaffold prior to or after implantation of the device. Alternatively, the isolated cells may be injected directly into the device and then cultured under conditions that promote proliferation and deposition of the appropriate biological matrix prior to *in vivo* implantation. In the preferred embodiment, the isolated cells are injected directly onto the scaffold in the device with no further *in vitro* culturing prior to *in vivo* implantation. Moreover, in the preferred embodiment the cells seeded in the implantable device are other than immune cells. Immune cells, as used herein, means cells that are part of acquired or innate immune response involving cells, including without limitation, polymorphonuclear neutrophils, eosinophils, basophils, lymphocytes, monocytes, T-cells, B-cells, plasma cells, antigen presenting cells, natural killer cells, macrophages and dentritic cells.

Cells that can be seeded or cultured on the scaffold of the current invention include, but are not limited to, bone marrow cells, mesenchymal stem cells, stromal cells, stem cells, embryonic stem cells, blood vessel cells, precursor cells derived from adipose tissue, bone marrow derived progenitor cells, intestinal cells, islets, Sertoli cells, beta cells, progenitors of islets, progenitors of beta cells, peripheral blood progenitor cells, stem cells isolated from adult tissue, and genetically transformed cells, or combination of the above cells. The cells can be seeded on the scaffold of the present invention for a short period of time, e.g. less than one day, just prior to implantation, or cultured for longer period, e.g. greater than one day, to allow for cell proliferation and matrix synthesis within the seeded scaffold prior to implantation.

The site of implantation is dependent on the diseased/injured tissue that requires treatment. For treatment of a disease such as diabetes mellitus (DM), the cell-seeded scaffold may be placed in a clinically convenient site such as the subcutaneous space or the omentum. In this particular case, the device of the present invention will act as a vehicle to entrap the administered islets in place after *in vivo* transplantation into an ectopic site.

The localization of the administered cells offers a significant advantage in treatment of DM, because the device of this invention forces cell-to-cell contact while providing a porous structure for transfer of nutrients and vascularization of the graft, which is essential for the proper long-term function of islets.

Previous attempts in direct transplantation of islets through injection into the portal circulation has proven inadequate in long-term treatment of diabetes. Furthermore, numerous methods of encapsulation of allogeneic or xenogeneic beta cells with biodegradable or nondegradable microspheres have failed to sustain long-term control of blood glucose levels. These failures have been attributed to inadequate vasculature and/or immune rejection of transplanted islets.

The failures can be circumvented by administering xenogeneic or allogeneic islets in combination with allogeneic or xenogeneic Sertoli cells that may aid in the survival of the islets and prevention of an immune response to the transplanted islets. Xenogeneic, allogeneic, or transformed Sertoli cells can protect themselves in the kidney capsule while immunoprotecting allogeneic or xenogeneic islets. The implantable device of the present invention circumvents the use of a clinical site that is difficult to access by co-seeding the islets and Sertoli cells in the device followed by transplantation into a clinically convenient site, such as the subcutaneous site. The scaffold allows for colocalization of these two cell types such that the Sertoli cells can immunoprotect islets that are in close vicinity, while providing an environment, which allows for formation of a vascularized bed.

Alternatively, the Sertoli cells may be cultured with the device before transplantation into an ectopic site, followed by administration of the islets into the graft site at some later point in time. In another embodiment, the islets and Sertoli cells may be injected into the implantable device at the same time prior to *in vivo* implantation. In yet another embodiment, the islets or Sertoli cells can be suspended in a synthetic polymer, such as polyethylene glycol, copolymers of polyethylene glycol and polylysine, hydrogels of alkyd polyesters, or a combination thereof, before injection into the lumen of the device. The cells may also be suspended in a biopolymer, such as hyaluronic acid, collagen, laminin, alginate, or a combination thereof, before injection into the lumen of the device.

In another alternative embodiment of the invention, the scaffold present in the lumen of the device or the shell of the device may be modified either through physical or chemical means to contain biological or synthetic factors that promote attachment, proliferation, differentiation, and matrix synthesis of targeted cell types. Furthermore, the bioactive factors may also comprise part of the matrix for controlled release of the factor to elicit a desired biological function. Another embodiment would include delivery of small molecules that affect the up-regulation of endogenous growth factors. Growth factors, extracellular matrix proteins, and biologically relevant peptide fragments that can be used with the matrices of the current invention include, but are not limited to, members of TGF-β family, including TGF-β1, 2, and 3, bone morphogenic proteins (BMP-2, -4, 6, -12, and -13), fibroblast growth factors-1 and -2, hepatocyte growth factor, platelet-derived growth factor-AA, and -BB, platelet rich plasma, insulin growth factor (IGF-I, II) growth differentiation factor (GDF-5, -6, -8, -10) vascular endothelial cell-derived growth factor (VEGF), pleiotrophin, endothelin, nicotinamide, glucagon like peptide-I and II, parathyroid hormone, tenascin-C, tropoelastin, thrombin-derived peptides, laminin, biological peptides containing cell- and heparin-binding domains of adhesive extracellular matrix proteins such as fibronectin and vitronectin and combinations thereof. The biological factors may be obtained either through a commercial source or isolated and purified from a tissue. Pharmaceutical agents that can be used with the implants of the current invention include, but are not limited to, anti-rejection agents, analgesics, anti-oxidants, anti-apoptotic agents such as Erythropoietin, anti-inflammatory agents such as antitumor necrosis factor α, anti-CD44, anti-CD3, anti-CD154, p38 kinase inhibitor, JAK-STAT inhibitors, anti-CD28, acetoaminophen, cytostatic agents such as Rapamycin, anti-IL2 agents, and combinations thereof.

The following examples illustrate the construction of an implantable device for implanting cells and cellular matter in mammals. Those skilled in the art will realize that these specific examples do not limit the scope of this invention and many alternative forms of an implantable device could also be generated within the scope of this invention.

### EXAMPLE 1: TEXTURED FIBROUS SCAFFOLD

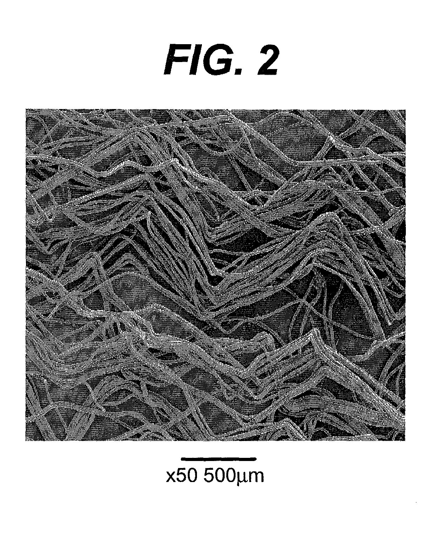

Fiber texturing was performed using a Techtex® HDC10 texturizer (Techniservice, Kennett Square, PA). Nine spools of 56 denier natural 90/10 glycolide-co-lactide (IV of about 1.1 deciliters per gram (dl/g) as measured in a 0.1 g/dl solution of hexafluoroisopropanol at 25 °C. The filaments had been drawn about 5X (original length compared to final length). The filaments were placed on the creel and combined into a single 504 denier tow by running the drawn yams together through a common eyelet. The individual yarn filament diameters were between 12-20 micron. A pretension of 5-7 grams was used for each yam by passing them through the gate tensioner. The large yarn tow was then passed over a heated godet with the separator roller (15 wraps) with the heated godet being set to a temperature of 130°C. This yam tow was then fed into the stuffer box by two crimper rolls. The clearance between the stuffer box and rollers was 0.012 inches and the temperature in the stuffer box was about 50°C. It should be noted that the box was not heated. Rather, the elevated temperature of 50°C was generated from the yarn, heated on the godet. Uniformity of crimp texture is maintained through accurate control of the crimped column height in the stuffer box. The column height control is provided by the optical sensor located in the stuffer box and signaling the take up winder inverter to speed up/slow down. The stuffer box optical sensor was set to hole no. 8 from the top of the box. After the stuffer box, the textured yarn tow passed through the gate tensioner set at 5 grams for combining and keeping all yams in the tow under the same tension. The crimped yam then passed the overfeed rolls to reduce high yarn tension prior to winding on the take up winder. The take up winder speed was set at 170 m/min. An image of the resulting textured fiber is shown in Figure 2.

### EXAMPLE 2: SHELL FORMATION

Tubes were formed from both poly(para-dioxanone) (PDO) and a copolymer of 35/65 epsilon-caprolactone/glycolide (CAP/GLY). The inherent viscosity (dl/g) of the PDO and CAP/GLY, as measured in a 0.1 g/dl solution of hexafluoroisopropanol (HFIP) 25°C, were 1.80 and 1.30, respectively. All shells were formed by extrusion using a ¾-inch Brabender single-screw extruder (C.W. Brabender® Instruments, Inc., So. Hackensack, NJ) under flowing nitrogen. Shells with several inner and outer dimensions were formed. Extrusion conditions for the extruded shells are shown in Table 1. Immediately following exit from the die, all shells were run through a 12-foot cooling trough filled with chilled water at a temperature of 5-10°C. For the CAP/GLY shells, short segments (~2-3 ft.) were cut and hung from one end at room temperature to allow solidification of the polymer.

**Table 1:**

| **Extrusion conditions** | | | | | | | | | | | | |
|---|---|---|---|---|---|---|---|---|---|---|---|---|
| Polymer | Die size Die x tip (mil) | T_{zone1} (°C) | T_{zone2} (°C) | T_{zone3} (°C) | T_{adapt.} (°C) | T_{die} (°C) | P_{block} (psi) | Pₐᵢᵣ (psi) | Screw speed (rpm) | Take-off (FTM ) | OD (mm ) | ID (mm ) |
| 35/65 CAP/G LY | 1 70 x 138 | 140 | 145 | 145 | 145 | 140 | 1900 | 0.1 | 12 | 20 | 2.0 | 1.5 |
| 35/65 CAP/G LY | 102 x 83 | 140 | 145 | 145 | 145 | 145 | 4480 | 0 | 4 | 18 | 1.03 | 0.83 |
| 35/65 CAP/G LY | 53 x40 | 140 | 145 | 145 | 145 | 140 | 4300 | 0.1 | 3 | 14 | 0.9 | 0.7 |
| 35/65 CAP/G LY | 56 X 40 | 140 | 145 | 150 | 150 | 150 | 2470 | 0.3 | 4 | 34 | 0.65 | 0.45 |
| PDO | 102 X 83 | 130 | 135 | 135 | 135 | 135 | 5000 | 0 | 5 | 20 | 1.03 | 0.83 |
| PDO | 102 X 83 | 145 | 150 | 150 | 150 | 150 | 3750 | 0 | 5 | 20 | 0.65 | 0.45 |

After extrusion, the shells were cut to the desired length (2-2.5 cm) using a razor blade. Membrane perforations were formed at Resonetics, Inc. (Nashua, NH) using an excimer laser (Lambda-Physik EMG201MSC Excimer Laser) operating at a wavelength of 193 nm. The laser was coupled to a Resonetics engineering workstation consisting of a mask projection imaging beam delivery system and a three-axis (XYtheta) computerized motion control system. Hole sizes ranging between 100 and 500 microns were formed through the membrane walls. Drilling parameters for the different shells are shown in Table 2.

**Table 2:**

| Laser drilling conditions | | | | |
|---|---|---|---|---|
| Polymer | OD/ID (mm/mm) | Fluence (J/cm²) | Pulse rate (Hz) | ~ Etch rate (µm/pulse) |
| 35/65 CAP/GLY | 2.0x1.5., 0.9x0.7 | 10 | 50 | 0.63 |
| 35/65 CAP/GLY | 2.0x1.5 | 3.5 | 50 | 0.56 |
| 35/65 CAP/GLY | 2.0x1.5 | 0.7 | 10 | 0.5 |
| 35/65 CAP/GLY | 1.03x0.83, 0.65x0.45 | 2 | 25 | 0.67 |
| PDO | 1.03x0.83, 0.65x0.45 | 2.6 | 50 | 0.5 |

### EXAMPLE 3: IMPLANTABLE TISSUE SCAFFOLD FORMATION WITH A FIBROUS SCAFFOLD

The textured fiber filling from Example 1 was placed inside the shells discussed in Example 2 as follows. Textured fiber was attached to a small needle or thin filament of wire and pulled through the shell. The fiber was cut to the length of the shell. Available porosity was calculated from the volume of the inner lumen of the shell, weight of textured yarn placed inside of the shell, and the density of the fibers used. Table 3 shows several of the construct geometries and resultant porosities.

**Table 3:**

| Absorbable constructs containing textured fiber. | | | | | | |
|---|---|---|---|---|---|---|
| Shell Composition | OD/ID/length (mm/mm/mm) | Hole diameter (µm) | # holes | Fiber weight (mg) | ~Percent porosity | Sample # |
| CAP/GLY | 2.0/1.5/25 | 300 | 20 | 12 | 80% | 1 |
| CAP/GLY | 2.0/1.5/20 | 300 | 16 | 10 | 80% | 2 |
| CAP/GLY | 2.0/1.5/20 | 300 | 12 | 10 | 80% | 3 |
| CAP/GLY | 2.0/1.5/20 | 300 | 8 | 10 | 80% | 4 |
| CAP/GLY | 2.0/1.5/20 | 300 | 4 | 10 | 80% | 5 |
| CAP/GLY | 2.0/1.5/20 | not applicable | 0 | 10 | 80% | 6 |
| CAP/GLY | 2.0/1.5/25 | 300 | 16 | 10 | 83% | 7 |
| CAP/GLY | 2.0/1.5/25 | 300 | 16 | 15 | 75% | 8 |
| CAP/GLY | 2.0/1.5/20 | 300 | 20 | 8 | 83% | 9 |
| CAP/GLY | 2.0/1.5/20 | 300 | 20 | 12 | 75% | 10 |
| CAP/GLY | 0.65/0.45/25 | 150 | 4 | 2 | 65% | 11 |
| CAP/GLY | 0.65/0.45/25 | 150 | 12 | 2 | 65% | 12 |
| CAP/GLY | 0.65/0.45/25 | 150 | 20 | 2 | 65% | 13 |
| PDO | 0.65/0.45/25 | 150 | 4 | 1.3 | 75% | 14 |
| PDO | 0.65/0.45/25 | 150 | 8 | 1.3 | 75% | 15 |
| PDO | 0.65/0.45/25 | 150 | 12 | 1.1 | 80% | 16 |
| PDO | 0.65/0.45/25 | 150 | 16 | 1.3 | 75% | 17 |

### EXAMPLE 4: IMPLANTABLE TISSUE SCAFFOLD FORMATION WITH A FOAM SCAFFOLD

This example describes the process for preparing devices with a foam scaffold inside the lumen of the device.

A solution of the polymer to be lyophilized into a foam scaffold was prepared. The polymer used to manufacture the foam component was a copolymer of 35 percent PCL and 65 percent PGA (35/65 PCL/PGA) produced by Birmingham Polymers Inc. (Birmingham, AL), with an I.V. of 1.79 dL/g, as measured in HFIP at 30°C. A 95/5 weight ratio of 1,4-dioxane/(35/65 PCL/PGA) was weighed out. The polymer and solvent were placed into a flask, which in turn was put into a water bath and stirred at 70°C for 5 hrs. The solution was filtered using an extraction thimble (extra coarse porosity, type ASTM 170-220 (EC)) and stored in a flask.

A laboratory scale lyophilizer, or freeze dryer, (Model Duradry, from FTS Kinetics, Stone Ridge, NY), was used to form the foam scaffold. A 35/65 epsilon-caprolactone/glycolide copolymer shell (1.5 mm ID) made following the process of Example 2 and cut to approximately 2 mm in length was placed in an aluminum mold. The polymer solution was carefully added into the lumen of the device.

The mold assembly was placed on the shelf of the lyophilizer and the freeze dry sequence begun. The freeze dry sequence used in this example was: 1) -17°C for 60 minutes; 2) -5°C for 60 minutes under vacuum 100 mT; 3) 5°C for 60 minutes under vacuum 20 mT; 4) 20°C for 60 minutes under vacuum 20 mT.

After the cycle was completed, the mold assembly was taken out of the freeze drier and allowed to degas in a vacuum hood for 2 to 3 hours. The tissue scaffold devices were stored under nitrogen.

The resulting devices contained foam scaffolds within the lumen of the device. Figure 3 is a scanning electron micrograph (SEM) of the cross-section of the implantable tissue scaffold device. The SEM clearly shows the lyophilized foam scaffold in the lumen of the device.

### EXAMPLE 5: IMPLANTABLE TISSUE SCAFFOLD FORMATION WITH A NONWOVEN SCAFFOLD

This example describes the process of preparing devices with a nonwoven scaffold inside the lumen of the device.

A nonwoven scaffold was prepared using a traditional needle punching technique as described below. A copolymer of PGA/PLA (90/10) was melt-extruded into continuous multifilament yarn by conventional methods of making yarn and subsequently oriented in order to increase strength, elongation, and energy required to rupture. The yams comprised filaments of approximately 20 microns in diameter. These yams were then cut and crimped into uniform 2-inch lengths to form 2-inch staple fiber.

A dry lay needle-punched nonwoven matrix was then prepared utilizing the 90/10 PGA/PLA copolymer staple fibers. The staple fibers were opened and carded on standard nonwoven machinery. The resulting mat was in the form of webbed staple fibers. The webbed staple fibers were needle-punched to form the dry lay needle-punched nonwoven scaffold.

The scaffold was rinsed in water followed by another incubation in ethanol to remove any residual chemicals or processing aids used during the manufacturing process.

A 35/65 epsilon-caprolactone/glycolide copolymer shell (approximately 2-mm ID) was made following the process of Example 2, cut to approximately 2 mm in length, and laser drilled with 16 holes (300 µm diameter).

The fabricated scaffolding mat was cut into 20 mm by 2 mm rectangles and placed inside the lumen of the shell. The weight of the nonwoven rectangles was 10 mg and the porosity was ~90percent. Figure 4 is a scanning electron micrograph (SEM) of the cross-section of the implantable tissue scaffold device. The SEM clearly shows the nonwoven scaffold inside the device.

### EXAMPLE 6: IMPLANTABLE TISSUE SCAFFOLDS WITH MAMMALIAN CELLS

This example illustrates seeding of Sertoli cells within the lumen of the device containing texturized fibers.

Sertoli cells were harvested from the testes of 9-12 day old male Balb/c mice. Testes were collected in Hank's balanced salt solution (HBSS), chopped into 1-mm pieces, and digested for 10 minutes at 37°C with collagenase (2.5 mg/ml; Sigma type V) in HBSS. The digest was rinsed three times with Ca²⁺/Mg²⁺-free HBSS containing 1 mmol/l EDTA and 0.5% bovine serum albumin (BSA), digested for 10 minutes at 37°C with trypsin (25 µg/ml Boehringer Mannheim) and Dnase (4 µg/ml, Boehringer Mannheim) in HBSS, followed by four washes in HBSS. The final cell pellet was resuspended in Hams-F10 (Gibco Life Technologies, Rockville, MD) supplemented with sodium bicarbonate, nicotinamide, D-glucose, L-glutamine, and calcium chloride dihydrate, passed through a 500 µm filter and cultured for 2 days in Ultra low cluster dishes (Coming Inc, Coming, NY) to allow aggregation of Sertoli cells.

Devices prepared as in Example 3 (Sample 2, with a CAP/GLY shell, 2 mm OD, 1.5 mm ID, 25 mm length, with 16 holes with diameters of 300 micron) were seeded with 1.2 million mice Sertoli cells and cultured for 3 weeks in Hams-F10 media supplemented with sodium bicarbonate, nicotinamide, D-glucose, L-glutamine, and calcium chloride dihydrate and Penicillin & Streptomycin. Following 3 weeks, the devices were fixed in 10% buffered formalin, embedded in paraffin and sectioned using a Zeiss Microtome. Cell distribution within the construct was assessed by hematoxylin & Eosin (H&E) staining.

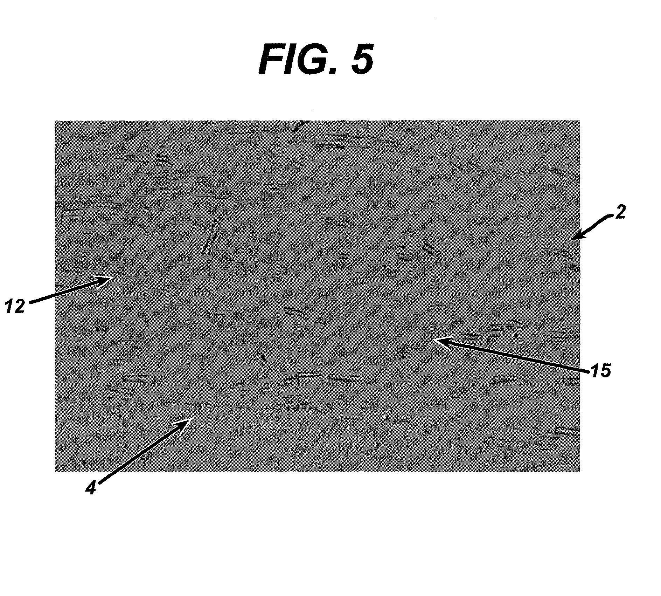

Figure 5 shows an H&E section of the construct with Sertoli cells 15.

## Claims

1. An implantable medical device suitable for implantation in a mammal for the treatment of a disease or the repair or regeneration of tissue of said mammal, comprising:
a biocompatible shell comprising an outer surface and an interior lumen; and
a biocompatible, porous scaffold comprising a plurality of at least one mammalian cell type other than an immune cell seeded therein, said scaffold being disposed within said interior lumen.

2. The device of claim 1 wherein said scaffold comprises a biocompatible fiber.

3. The device of claim 2 wherein said scaffold comprises a textured yarn.

4. The device of claim 3 wherein said textured yarn is selected from the group consisting of bulked yarns, coil yarns, core bulked yarns, crinkle yarns, entangled yarns, modified stretch yarns, nontorqued yarns, set yarns, stretch yarns and torqued yarns.

5. The device of claim 2 wherein said scaffold comprises nonwoven fibers.

6. The device of claim 1 wherein said scaffold comprises a biocompatible foam.

7. The device of claim 6 wherein said foam comprises a lyophilized foam.

8. The device of claim 1 wherein said device is bioabsorbable.

9. The device of claim 8 comprising a polymer selected from the group consisting of aliphatic polyesters, poly(amino acids), copoly(ether-esters), polyalkylenes oxalates, polyamides, tyrosine derived polycarbonates, poly(iminocarbonates), polyorthoesters, polyoxaesters, polyamidoesters, polyoxaesters containing amine groups, poly(anhydrides), polyphosphazenes and biopolymers.

10. The device of claim 9 comprising an aliphatic polyester.

11. The device of claim 1 wherein said shell comprises an aliphatic polyester selected from the group consisting of homopolymers and copolymers of lactide, glycolide, epsilon-caprolactone, para-dioxanone and trimethylene carbonate.

12. The device of claim 1 wherein said scaffold comprises an aliphatic polyester selected from the group consisting of homopolymers and copolymers of lactide, glycolide, epsilon-caprolactone, para-dioxanone and trimethylene carbonate.

13. The device of claim 1 wherein said shell comprises poly(para-dioxanone) and said scaffold comprises a copolymer of about 90 weight percent glycolide and about 10 weight percent lactide.

14. The device of claim 1 wherein said biocompatible scaffold comprises a biocompatible foam scaffold, wherein said foam comprises a copolymer of about 35 weight percent epsilon-caprolactone and about 65 weight percent glycolide.

15. The device of claim 14 wherein said biocompatible foam scaffold comprises a lyophilized foam.

16. The device of claim 1 wherein said mammalian cells are selected from the group consisting of bone marrow cells, mesenchymal stem cells, stromal cells, stem cells, embryonic stem cells, blood vessel cells, precursor cells derived from adipose tissue, bone marrow derived progenitor cells, intestinal cells, islets, Sertoli cells, beta cells, progenitors of islets, progenitors of beta cells, peripheral blood progenitor cells, stem cells isolated from adult tissue and genetically transformed cells.

17. The device of claim 16 wherein said mammalian cells comprise islet and Sertoli cells.

18. The device of claim 1 further comprising a material selected from the group consisting of growth factors, extracellular matrix proteins, biologically relevant peptide fragments, hepatocyte growth factor, platelet-derived growth factors, platelet rich plasma, insulin growth factor, growth differentiation factor, vascular endothelial cell-derived growth factor, nicotinamide, glucagon like peptides, tenascin-C, laminin, anti-rejection agents, analgesics, anti-oxidants, anti-apoptotic agents anti-inflammatory agents and cytostatic agents.

19. The device of claim 18 wherein said material comprises an anti-inflammatory agent.

20. The device of claim 1 wherein said scaffold is seeded with said cells prior to implantation of said device in said mammal.

21. The device of claim 1 wherein said scaffold is seeded with said cells after implantation of said device in said mammal.

22. The device of claim 1 wherein said outer surface of said device comprises pores, wherein the cross-sectional area of said pores comprises up to about 95 percent of said outer surface.

23. The device of claim 22 wherein the diameter of said pores is from about 0.1 to about 500 microns.

24. The device of any one of claims 1 to 23 for use in treating a disease in a mammal.
